# EUROPÄISCHE PATENTSCHRIFT

(11) **EP 0 483 567 B1**
(45) Veröffentlichungstag und Bekanntmachung des Hinweises auf die Patenterteilung: **20.12.1995**
(21) Anmeldenummer: 91117331.8
(22) Anmeldetag: 11.10.1991
(51) Int. Cl.: A61F 11/00, A61B 17/00, G01N 1/06

(54) **Medizinische Schneidvorrichtung**
Medical cutting device
Dispositif médical à couper

(30) Priorität: 31.10.1990 DE 4034615
(43) Veröffentlichungstag der Anmeldung: 06.05.1992
(73) Patentinhaber: Kurz, Heinz, D-72144 Dusslingen (DE)
(72) Erfinder: Kurz, Heinz, D-72144 Dusslingen (DE)
(74) Vertreter: Möbus, Rudolf, Dipl.-Ing.

(56) Entgegenhaltungen:
- GB-A- 1 179 927
- SU-A- 927 245
- SU-A- 1 355 894
- SU-A- 1 487 903
- US-A- 3 261 600
- US-A- 4 747 331

## Beschreibung

Die Erfindung betrifft eine medizinische Schneidvorrichtung zur Herstellung dünner Knorpelscheiben.

Bei vielen chirurgischen Eingriffen werden dünne Knorpelscheiben benötigt, beispielsweise im Mittelohrbereich zum Abdecken eines Ohrimplantates, zum Wiederaufbau der Gehörgangshinterwand oder zur plastischen Versorgung eines Trommelfelldefekts. Von Ohrmuschel, Tragus oder Nasenseptum entnommene Knorpelstücke, wie sie auch bei dem aus der SU-A-1487903 bekannten Verfahren verwendet werden, sind für diesen Zweck zu dick. Der Erfindung liegt daher die Aufgabe zugrunde, aus entnommenen Knorpelstücken dünne Knorpelscheiben rasch und sicher herzustellen.

Die gestellte Aufgabe läßt sich erfindungsgemäß mit einer Schneidvorrichtung lösen, die durch einen einen Deckel aufweisenden Vorrichtungskörper gekennzeichnet ist, auf dessen Oberseite eine zu einer Stirnseite des Körpers hin offene, nach oben durch den Deckel verschließbare Vertiefung ausgebildet ist, und der in den seitlichen Begrenzungsstegen der Vertiefung einen von der Stirnseite des Körpers her geführten, mit einem vorgegebenen Abstand parallel zur Grundfläche der Vertiefung verlaufenden Führungsschlitz für eine Schneidklinge, insbesondere Rasierklinge, aufweist.

Bei der erfindungsgemäß ausgebildeten medizinischen Schneidvorrichtung wird das Knorpelexzidat bei abgenommenem Deckel in die Vertiefung gelegt, anschließend der Deckel aufgesetzt und dann durch Einbringen der Rasierklinge in den Führungsschlitz eine Knorpelscheibe von beispielsweise 0,5 mm Dicke hergestellt. Durch Einlegen maßgenauer Distanzblättchen in die Vertiefung können noch dünnere Knorpelscheiben erhalten werden. Die dünnen Knorpelscheiben werden rasch und mit wenigen Handgriffen erhalten, wodurch die Operationszeit verkürzt wird.

Der Deckel kann auf seiner Abdeckfläche vorteilhafterweise einen teilweise in die Vertiefung des Vorrichtungskörpers vorstehenden, aber mit Abstand vor dem Führungsschlitz endenden Vorsprung aufweisen. Dadurch wird die Halterung eines Knorpelexzidats in der Vorrichtung begünstigt. Die Vertiefung ist zweckmäßig mit schrägen Randflächen und mit einer rechteckigen Grundfläche mit abgerundeten Innenecken versehen.

Es hat sich als vorteilhaft erwiesen, den Vorrichtungskörper und den Deckel jeweils von der mit dem Führungsschlitz versehenen Stirnseite des Körpers weg zu einem von beiden Teilen gebildeten Griffteil zu verlängern und hierbei Vorrichtungskörper und Deckel mit einem deckungsgleichen Grundriß zu versehen. Da durch den in die Vertiefung ragenden Deckelvorsprung eine teilweise Zentrierung des Deckels auf dem Körper erfolgt, genügt zur Befestigung des Deckels auf dem Vorrichtungskörper eine einzige Spannschraube. Sie erbringt den Vorteil, daß bei gelockerter Spannschraube der Deckel gegenüber dem Vorrichtungskörper verdreht werden kann, wodurch auf rasche Weise nach der Ausführung des Schnittes die Vertiefung zur Entnahme der Knorpelscheibe geöffnet werden kann. Mittels am Vorrichtungskörper oder dem Deckel ausgebildeter Paßvorsprünge, die in der Schließstellung der Vorrichtung in Paßöffnungen des anderen Teiles eingreifen, kann eine zusätzliche Zentrierung von Deckel und Vorrichtungskörper in der Schließstellung der Vorrichtung erreicht werden.

Nachfolgend wird ein Ausführungsbeispiel einer erfindungsgemäß ausgebildeten Schneidvorrichtung anhand der beiliegenden Zeichnung näher erläutert.

Im einzelnen zeigen:
- Fig. 1: eine perspektivische Darstellung der Schneidvorrichtung mit durch Verdrehen des Deckels gegenüber dem Vorrichtungskörper geöffneter Vertiefung;
- Fig. 2: eine Ansicht der Oberseite des Vorrichtungskörpers;
- Fig. 3: eine Seitenansicht des Vorrichtungskörpers;
- Fig. 4: eine Ansicht der Deckfläche des Deckels der Vorrichtung;
- Fig. 5: eine Seitenansicht des Deckels.

Die Schneidvorrichtung 10 besteht aus einem Vorrichtungskörper 11 und einem Deckel 12, die beide mit dem gleichen Grundriß und auch etwa massegleich aus Metall gefertigt sind. Vorrichtungskörper 11 und Deckel 12 bilden zusammen einen Griffteil und sind nach einer Stirnseite 11.1 und 12.1 hin verschmälert. In diesem verschmälerten Bereich weist der Vorrichtungskörper 11 eine rechteckige Vertiefung 13 auf, die bis zur Stirnseite 11.1 reicht und beidseitig von einem Randsteg 14 und 15 begrenzt ist. Die Randstege 14 und 15 bilden schräge Randflächen 14.1, 15.1. Die inneren Ecken 16 der Vertiefung sind abgerundet.

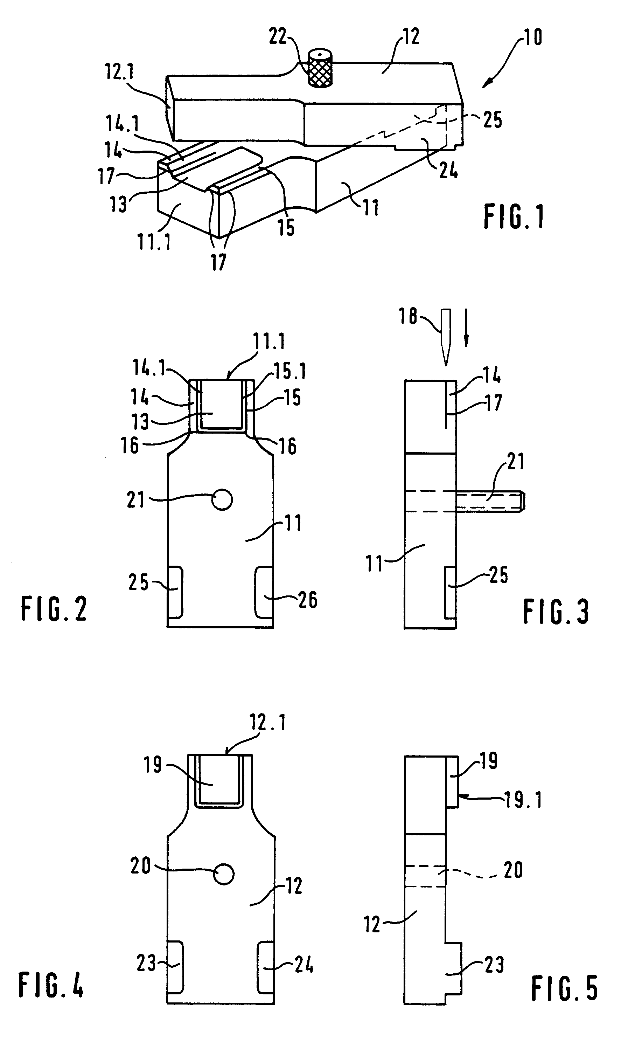

In den beiden Randstegen 14 und 15 sind in einer gemeinsamen und parallel zur Grundfläche der Vertiefung 13 verlaufenden Ebene von der Stirnseite 11.1 des Vorrichtungskörpers 11 her Führungsschlitze 17 ausgebildet, in welche gemäß Fig. 3 eine flache Schneidklinge, insbesondere eine Rasierklinge 18, einführbar ist. Die Führungsschlitze 17 verlaufen in einem vorgegebenen Abstand vom Grund der Vertiefung 13. Dieser Abstand kann durch Auflegen nicht dargestellter Distanzblättchen auf den Grund der Vertiefung um ebenfalls festgelegte Werte verringert werden. Dieser Abstand bestimmt die Dicke der Knorpelscheiben, die aus einem in die Vertiefung 13 eingelegten Knorpelexzidat gefertigt werden sollen.

Die Fig. 4 und 5 zeigen den auf den Vorrichtungskörper 11 aufsetzbaren Deckel 12. Er weist auf seiner aus Fig. 4 ersichtlichen Abdeckfläche einen an die Form der Vertiefung 13 des Vorrichtungskörpers 11 angepaßten Vorsprung 19 auf, der in einer ebenen Abschlußfläche 19.1 endet. Die Höhe der Erhebung 19 ist so bemessen, daß sie bei aufgesetztem Deckel 12 mit Abstand vor den Führungsschlitzen 17 des Vorrichtungskörpers 11 endet. Der Deckel 12 ist mit einer Durchgangsbohrung 20 für einen im Vorrichtungskörper 11 verankerten Gewindebolzen 21 versehen, auf welchen eine aus Fig. 1 ersichtliche Haltemutter 22 aufschraubbar ist. Die Zentrierung des Deckels 12 auf dem Vorrichtungskörper 11 in der Schließstellung der Schneidvorrichtung 10 erfolgt einerseits durch die Erhebung 13 und anderseits durch zwei am Griffteilende des Deckels über die Abdeckfläche vorstehende Randstege 23 und 24, welche in passende Ausnehmungen 25 und 26 des Vorrichtungsteiles ragen.

Es versteht sich, daß sich die Größe der Schneidvorrichtung und die Abmessungen ihrer Vertiefung 13 an bestimmte Anwendungsfälle, insbesondere auch an bestimmte Formen von Knorpelexzidaten, anpassen lassen.

## Patentansprüche

1. Medizinische Schneidvorrichtung zur Herstellung dünner Knorpelscheiben, gekennzeichnet durch einen einen Deckel (12) aufweisenden Vorrichtungskörper (11), auf dessen Oberseite eine zu einer Stirnseite (11.1) des Körpers (11) hin offene, nach oben durch den Deckel (12) verschließbare Vertiefung (13) ausgebildet ist, und der in den seitlichen Begrenzungsstegen (14, 15) der Vertiefung (13) einen von der Stirnseite (11.1) des Körpers (11) her geführten, mit einem vorgegebenen Abstand parallel zur Grundfläche der Vertiefung (13) verlaufenden Führungsschlitz (17) für eine Schneidklinge (18), insbesondere Rasierklinge, aufweist.

2. Schneidvorrichtung nach Anspruch 1, dadurch gekennzeichnet, daß der Deckel (12) auf seiner Abdeckfläche einen teilweise in die Vertiefung (13) des Vorrichtungskörpers (11) vorstehenden, aber mit Abstand vor dem Führungsschlitz (17) endenden Vorsprung (19) aufweist.

3. Schneidvorrichtung nach Anspruch 1 oder 2, dadurch gekennzeichnet, daß die Vertiefung (13) schräge Randflächen (14.1, 15.1) und eine rechteckige Grundfläche mit abgerundeten Innenecken (16) aufweist.

4. Schneidvorrichtung nach einem der Ansprüche 1 bis 3, dadurch gekennzeichnet, daß der Deckel (12) mittels einer Schraube oder Mutter (22) am Vorrichtungskörper (11) lösbar befestigbar ist.

5. Schneidvorrichtung nach einem der Ansprüche 1 bis 4, dadurch gekennzeichnet, daß Vorrichtungskörper (11) und Deckel (12) jeweils von der mit dem Führungsschlitz (17) versehenen Stirnseite (11.1) des Körpers (11) weg zu einem Griffteil verlängert sind.

6. Schneidvorrichtung nach einem der Ansprüche 1 bis 5, dadurch gekennzeichnet, daß Vorrichtungskörper (11) und Deckel (12) einen deckungsgleichen Grundriß aufweisen.

7. Schneidvorrichtung nach einem der Ansprüche 1 bis 6, dadurch gekennzeichnet, daß der Vorrichtungskörper (11) oder der Deckel (12) Paßvorsprünge (23, 24) aufweist, die in der Schließstellung der Vorrichtung in Paßöffnungen (25, 26) des anderen Teiles eingreifen.

## Claims

1. A medical cutting device for producing thin cartilaginous sections, characterised by a device body (11) which has a cover (12) and on the upper face of which an indentation (13) is formed which is open towards an end face (11.1) of the body (11) and which can be closed at the top by the cover (12), and which device body has a guide slot (17) for a cutting blade (18), particularly a razor blade, in the lateral delimiting ribs (14, 15) of the indentation (13), which guide slot extends at a predetermined distance parallel to the base of the indentation (13) and is led from the end face (11.1) of the body (11).

2. A cutting device according to claim 1, characterised in that the cover (12) has a projection (19) on its covering face, which projection partly projects into the indentation (13) of the device body (11) but ends at a distance in front of the guide slot (17).

3. A cutting device according to claim 1 or 2, characterised in that the indentation (13) has bevelled edge faces (14.1, 15.1) and a rectangular base with rounded-off internal corners (16).

4. A cutting device according to any one of claims 1 to 3, characterised in that the cover (12) can be detachably secured to the device body (11) by means of a screw or nut (22).

5. A cutting device according to any one of claims 1 to 4, characterised in that the device body (11) and the cover (12) are each extended away from the end face (11.1) of the body (11) which is provided with the guide slot (17) to form a handle part.

6. A cutting device according to any one of claims 1 to 5, characterised in that the device body (11) and the cover (12) have a congruent horizontal projection.

7. A cutting device according to any one of claims 1 to 6, characterised in that the device body (11) or the cover (12) has fitting projections (23, 24) which engage in fitting openings (25, 26) of the other part when the device is in its closed position.

## Revendications

1. Dispositif de coupe médical pour produire des rondelles de cartilage minces, caractérisé par un corps de dispositif (11) présentant un couvercle (12), sur le côté supérieur duquel est formé un renfoncement (13) ouvert vers un côté frontal (11.1) du corps (11), pouvant être fermé vers le haut par le couvercle (12) et qui présente dans les entretoises de limitation latérales (14, 15) du renfoncement (13) une fente de guidage (17) pour lame de coupe (18), en particulier une lame de rasoir ménagée depuis le côté frontal (11.1) du corps (11), se développant à une distance prédéterminée et parallèlement à la surface de base du renfoncement (13).

2. Dispositif de coupe selon la revendication 1, caractérisé en ce que le couvercle (12) présente sur sa surface de recouvrement une saillie (19) dépassant partiellement dans le renfoncement (13) du corps de dispositif (11), mais se terminant avec écartement devant la fente de guidage (17).

3. Dispositif de coupe selon la revendication 1 ou 2, caractérisé en ce que le renfoncement (13) présente des surfaces de bord obliques (14.1, 15.1) et une surface de base rectangulaire avec des coins intérieurs arrondis (16).

4. Dispositif de coupe selon l'une des revendications 1 à 3, caractérisé en ce que le couvercle (12) peut être fixé amovible au moyen d'une vis ou d'un écrou (22) au corps de dispositif (11).

5. Dispositif de coupe selon l'une des revendications 1 à 4, caractérisé en ce que le corps de dispositif (11) et le couvercle (12) sont prolongés respectivement depuis le côté frontal (11.1) du corps (11) muni de la fente de guidage (17) en une pièce de poignée.

6. Dispositif de coupe selon l'une des revendications 1 à 5, caractérisé en ce que le corps de dispositif (11) et le couvercle (12) présentent une vue en plan congruente.

7. Dispositif de coupe selon l'une des revendications 1 à 6, caractérisé en ce que le corps de dispositif (11) ou le couvercle (12) présente des saillies d'adaptation (23, 24) qui dans la position fermée du dispositif s'engagent dans des ouvertures d'adaptation (25, 26) de l'autre pièce.
